# EUROPEAN PATENT APPLICATION

(11) **EP 1 537 871 A1**
(43) Date of publication of application: **08.06.2005**
(21) Application number: 03293028.1
(22) Date of filing: 04.12.2003
(51) Int. Cl.: A61K 31/727, A61P 35/00

(54) **Enoxaparin for the treatment of cancer**

(71) Applicant: Aventis Pharma S.A., 92160 Antony (FR)
(72) Inventor: Uzan, André, 75116 Paris (FR); Shukla, Umesh A., Belle Mead, NJ 08502 (US); Samuel, Rita, Basking Ridge, NJ 07920 (US); Toro-Figuerola, Luis, Belle Mead, NJ 08502 (US)
(74) Representative: Rousseau, Pierrick Edouard

(57) **Abstract**

The present invention relates to the use of enoxaparin for treating a disease linked to the modulation of heparanase activity, in particular cancers like breast, lung, prostate, colon or pancreatic cancers.

## Description

The present invention relates to the use of enoxaparin for treating a disease linked to the modulation of heparanase activity.

Enoxaparin (Lovenox^{TM}, Clexane^{TM}) is a low-molecular-weight heparin, which is marketed for the prophylactic treatment of venous thromboembolic disease in moderate- or high-risk surgery, the prevention of coagulation in the extracorporeal circulation system during hemodialysis, the treatment of constituted deep venous thromboses and, in combination with aspirin, for the treatment of unstable angina and of acute non-Q wave myocardial infarction. Enoxaparin is also useful in the prevention and/or the treatment of trauma of the central nervous system (WO 98/53833) and of cerebral edemas (WO 98/53834). Enoxaparin is also useful in the prevention and/or treatment of motoneuron diseases (WO 00/35462), and for the treatment of cerebral ischemia (WO 01/49298).

Patients with cancer have increased risk of venous thrombo-embolism (VTE), similar to the risk after major orthopedic surgery. Thrombosis is the second leading cause of death in patients with cancer (Green K.B.et al., Hematol. Oncol. Clin. North Am. 10:499-530, 1996). Patients with cancer are often included in clinical studies with heparins (unfractionated heparin (UFH) and low molecular weight heparin (LMWH)) for the treatment or prevention of deep vein thrombosis (DVT) or pulmonary embolism (PE). Several clinical studies reported improved survival in cancer patients with UFH (Lebeau B. et al., Cancer 74:38-45, 1994; Zacharski L. et al., Thromb. Haemost. 80:10-23, 1998), and with LMWH (Bergqvist D. et al., N. Engl. J. Med. 346:975-980, 2002; von Tempelhoff G., Int. J. Oncol. 10:815-824, 2000). In a small Phase II study in 15 patients of docetaxel plus enoxaparin in chemotherapy-naïve patients with metastatic non-small cell lung cancer, enoxoparin is used to prevent thrombosis events in cancer patients when treated with docetaxel and is reported to significantly decrease angiogenic protein TGF-β1 levels in patients (Robert F. et al., Lung Cancer 42:237-245, 2003). Thus these studies suggested a role of heparins in cancer therapy related to the prevention and/or treatment of venous thrombosis and pulmonary embolism.

The pathophysiology of malignancy is complex and multifactorial. Among the processes involved : inflammation, thrombin and fibrin formation, cancer cell proliferation, angiogenesis, migration of cancer cells (metastasis) are recognized as major components of the disease.

Cancer cell migration (metastasis) is dependent notably upon extra cellular matrix degradation. Heparanases, a family of endoglycosidases, provokes matrix degradation promoting cell invasion. Heparanase activity is involved in different biological processes like the extravasation of inflammatory cells and also of tumor cells during metastasis. Heparanase activity is found in a variety of normal and malignant cells and tissues among which are endothelial cells, platelets, mast cells, neutrophils, macrophages, T and B lymphocytes, lymphoma, melanoma, and carcinoma cells. It is described distinct heparanases from human platelets, mouse macrophages and melanoma cells, which represent the product of one single gene (Vlodavsky I. et al., Nat. Med. 7:793-802, 1999).

The usual substrate of this enzyme is heparan sulfate, with high substrate specificity. Heparan sulfate is cleaved by heparanase and this degradation has multiple pathological consequences. As a matter of fact, heparan sulfate plays a central role in normal and pathological processes among which are tissue repair, inflammation, autoimmunity, tumor growth and metastasis. Enzymatic degradation of heparan sulfate is likely to be involved in development of inflammation and cancer metastasis (Hulette M.D. et al., Nat. Med. 7: 803-809, 1999).

Some documents discuss the inhibition of heparanase activity in the presence of different heparin species (Vlodavski I. et al., Adv. Exp.Med. Biol. 313:317-27, 1992; Parish C.R. et al., Int. J. Cancer 40: 511-518, 1987; Bitan. M. et al, Isr. J. Med Sci. 31:106-118, 1995). Heparin is considered as heparanase inhibitor and its inhibitory activity is linked to an immunomodulating action (Gorski A et al., FASEB J. 5: 2287-2291, 1991). This does not at all encourage the use of enoxaparin as an inhibitor of heparanase. It is well known, compared to unfractionated heparin, that enoxaparin has different activity. Enoxaparin is a mixture of fragments ranging from 600 to 14,000 daltons whereas unfractionated Heparin is a mixture of fragments ranging from 5,000 to 30,000 daltons. The fragments between 600 and 5,000 daltons are negligible in the unfractionated Heparin. As these fragments between 600 and 5,000 daltons represent more than 60 % of enoxaparin.

In the article Pacheco (J. Dermatol. Treat. 12: 123-126, 2001), it is quoted that a "Low-molecular-weight heparin (enoxaparin) has been shown to inhibit expression of heparanase" for the treatment of Lichen planus. However, this information has to be read in connection with the fact that said inhibition is deemed to happen because CD4+ lymphocytes produce endoglycosidase (heparanase), which allows them to penetrate the endothelial basal lamina. In addition the authors only demonstrate that Lichen planus is efficiently treated when enoxaparin is administered to the patient in need thereof. The authors do not also demonstrate the causes of this effect, e.g. if CD4+ lymphocytes cannot attack the epidermis, if CD4+ lymphocytes are no more "activated", if CD4+ lymphocytes produce heparanase that is inhibited by enoxaparin.

In another aspect, the article Amirkhosravi et al. (J. Thromb. Haemostasis 1:1972-1976, 2003) tinzaparin (a low molecular weight heparin) was reported to inhibit metastasis in lung tumor in the mice in the B 16 melanoma model, due to its effective releasing of endothelial Tissue Factor Pathway Inhibitor (TFPI). In addition, enoxaparin has a great variability of activity compared to other LMWHs. Like most LMWHs, enoxaparin comes from unfractionated heparin source material. LMWH is manufactured by breakdown of larger unfractionated heparin chains into smaller ones through varying processes of chemical or enzymatic depolymerization. Each LMWH manufacturer utilizes a distinct process of depolymerization. This results in LMWHs with distinct chemical structures and therefore, differing pharmacological activity. An article of Fareed J. et al. (Annals New York Academy Sciences 556, 1989) shows that all LMWHs are not equivalent as well from the biochemical point of view as pharmacological.

Patients with cancer have limited treatment options and response to these limited treatments is less than optimal, notably for the inhibition of metastasis occurrence. There is still a need to provide new drug that can enhance the response to cancer treatments, the inhibition of metastasis, and improving penetration of cytotoxic agent inside the tumor cells. This is expected to result in improved survival and better quality of life for these patients.

It has now been found surprisingly that enoxaparin modulates the heparanase activity involved in diseases. In particular it has been found that enoxaparin inhibits heparanase activity.

In one embodiment, the present invention relates to the use of enoxaparin for treating a disease linked to modulation of heparanase activity, except when the said disease is Lichen planus.

In another embodiment, the invention relates to the use of enoxaparin in treating a disease in a mammal in which heparanase activity contributes to the pathology and/or symptoms of the disease except when said disease is Lichen planus.

In another embodiment, the invention relates to the use of enoxaparin in treating a disease in a mammal in which heparanase activity contributes to the pathology and/or symptoms of the disease, except when said disease is an autoimmune disease.

The present invention relates also to the use of enoxaparin for treating a disease linked to modulation of heparanase activity, except when the said disease is an autoimmune disease.

For example, the said autoimmune disease is selected from the group including, but not limited, to multiple sclerosis, autoimmune encephalomyelitis and Lichen planus. In particular, the said autoimmune disease is Lichen planus.

The invention relates also to the use of enoxaparin in treating a disease in a mammal in which heparanase activity contributes to the pathology and/or symptoms of the disease, except when said disease is mainly linked to inflammation of the central nervous system.

In another embodiment, the present invention relates to the use of enoxaparin for the manufacture of a medicament for treatment of a disease linked to modulation of heparanase activity, except when said disease is Lichen planus.

In another embodiment, the present invention relates to the use of enoxaparin for the manufacture of a medicament for treatment of a disease linked to modulation of heparanase activity, except when said disease is an autoimmune disease.

In another embodiment, the present invention relates more particularly to the above-mentioned use of enoxaparin, wherein disease is linked to the inhibition of heparanase.

The above-mentioned disease is preferably cancer.

The said cancer is selected from the group including, but not limited, to breast cancer, lung cancer, prostate cancer, colon cancer or pancreatic cancer.

A particular preferred cancer is breast cancer.

In another embodiment, the present invention relates more preferably to the above-mentioned use, wherein enoxaparin is in combination with one or more chemotherapeutical agent.

The said chemotherapeutical agent is selected from the group including, but not limited, to docetaxel (INN), paclitaxel (INN), cyclophosphamide (INN), or anthracyclines.

A preferred anthracycline is particularly doxorubicin (INN).

A preferred anthracycline is particularly epirubicin (INN).

A particular preferred chemotherapeutical agent is docetaxel.

In another embodiment, the present invention relates more preferably to the above-mentioned use, wherein enoxaparin is in combination with docetaxel and doxorubicin.

For example the present invention relates also to the use of enoxaparin in combination with docetaxel, doxorubicin, and cyclophosphamide.

Reference to the preferred embodiments set forth above is meant to include all combinations of particular and preferred groups.

In another embodiment, the present invention relates more particularly to the above-mentioned use, wherein enoxaparin is in combination with one or more chemotherapeutical agent, wherein said combination is a combined preparation for simultaneous, separate or sequential use.

In another particular embodiment, the present invention relates the above-mentioned use of enoxaparin, wherein enoxaparin reduces metastasis occurrence.

In another embodiment, the invention relates to a method for treating a disease linked to modulation of heparanase activity, except wherein said disease is Lichen planus, which method comprises administering to an animal a therapeutically effective amount of enoxaparin or a combination of enoxaparin and one or more chemotherapeutical agent or a pharmaceutically acceptable salt thereof, either simultaneously or separately or sequentially over time

In another embodiment, the invention relates to a method for treating a disease linked to modulation of heparanase activity, except when said disease is an autoimmune disease, which method comprises administering to an animal a therapeutically effective amount of enoxaparin or a combination of enoxaparin and one or more chemotherapeutical agent or a pharmaceutically salt thereof, either simultaneously or separately or sequentially over time.

In another embodiment, the present invention relates more particularly to the above-mentioned method, wherein disease is linked to the inhibition of heparanase.

In another embodiment, the invention relates to a method to potentiate the action of one or more chemotherapeutical agent, which comprises simultaneous, separate or sequential administration of enoxaparin.

The above-mentioned chemotherapeutical agent is selected from the group including, but not limited, to docetaxel, paclitaxel , cyclophosphamide, or anthracyclines.

A preferred anthracycline are particularly doxorubicin.

A preferred anthracycline are particularly epirubicin.

A particular preferred chemotherapeutical agent is docetaxel.

The present invention relates more preferably to the above-mentioned method, wherein enoxaparin is in combination with docetaxel and doxorubicin.

For example the present invention relates also to the above-mentioned method, wherein enoxaparin is in combination with docetaxel, doxorubicin, and cyclophosphamide.

Reference to the preferred embodiments set forth above is meant to include all combinations of particular and preferred groups.

The above-mentioned disease is preferably cancer.

The said cancer is selected from the group including, but not limited, to breast cancer, lung cancer, prostate cancer, colon cancer or pancreatic cancer.

A particular preferred cancer is breast cancer.

The evaluation of enoxaparin with respect to its ability to inhibit heparanases was performed as follow.

Radiolabelled heparin/heparan sulfate (HS) is degraded by heparanases, resulting in low molecular weight fragments of HS that can be measured by gel permeation chromatography (FPLC) and liquid scintillation counting of fractions.

Unfractionated heparin (sodium salt) from Porcine intestinal mucosa (grade Ia, 183 USP/mg) was obtained from Sigma Biochemicals (Deisenhofen, Germany). Heparitinase (HP lyase (EC 4.2.2.8)) was purchased from Seikagaku, (Tokyo, Japan). TSK 4000 was from Toso Haas and Sepharose Q columns equipped with guard columns were obtained from Pharmacia/LKB (Freiburg, Germany).

A human cervix fibroblast cell line was used to prepare 35-S labelled heparan sulfate (proteoglycans) by metabolic labelling. This cell line has been shown to produce relative large amounts of different heparan sulfate proteoglycans (HS-PG), such as syndecans and glypican (Drzeniek et al., Blood 93:2884-2897, 1999).

Labelling is achieved by incubation of the cells at a cell density of approx. 1x10-6 cells/ ml with 33µCi/ml 35-S-sulfate in tissue culture medium for 24 hours. Thereafter supernatants were harvested and protease inhibitor PMSF (phenylmethylsulfonyl fluoride) (1mmol/L) was added. Heparan sulfate proteoglycans (HS-PG) were purified by anion exchange chromatography on Sepharose Q, removal of chondroitin sulfate/dermatan sulfate-proteoglycans was not necessary, as the sample contained a relative high amount of heparan sulfate proteoglycans and due to the specificity of the enzyme heparanase.

Heparanase was prepared from human peripheal blood leukocytes (PBL, buffy coats) and enriched for polymorphonuclear cells (PMN) by ficoll-gradient procedures. Isolated PMN were adjusted to 2,5 x 10-7 cells/ml and incubated for 1 hours at 4°C. Thereafter, supernatants containing the heparanase were harvested, adjusted to ph 6.2 (20 mM citrate-phosphate buffer) and used immediately or stored frozen in aliquots at -20°C.

200 µl of 35-S-labelled heparan sulfate (proteoglycans) adjusted to about 2200 dpm/ml is incubated at 37°C for 18 hours with 1 µl of the heparanase containing PMN supernatant. Thereafter, 200 µl of the sample were applied to a TSK 4000 gel permeation chromatography column (FPLC), fractions were collected and analysed by liquid scintillation counting. Degradation was calculated according to the following formula:

% degradation=[[∑ counts (dpm) tract. 20-33 (HEP) -∑ counts (dpm) fract. 20-33 (CONT)] / [ total counts (dpm) fract. 12-33 (CONT)]] x 100

e.g. the sum of counts (dpm) in fractions 20-33 of the sample after heparanase treatment, from this sum the background counts (dpm) (fractions 20-33) of the control sample was substracted. This sum was divided by the total counts (fractions 12-33) applied to the column, to calculate the % degradation. Correction factors were used to normalize total counts of different chromatographies to 2200 counts/dpm. Results are given as percent degradation. In the inhibition experiments, the degradation of the control sample (with heparanase) was set to 100 % (degradation) and the % inhibition values were calculated accordingly. A correction for sulfatase activity was not necessary, as no sulfatase activity could be detected.

The following inhibitors of heparanase, unfractioned heparin (UF-H) and enoxaparin (WSD 3014), were tested in the assay described above at three different concentrations. The comparison was performed on a weight basis. Data are expressed as percent inhibition of heparanase activity.

The results obtained are as follows.

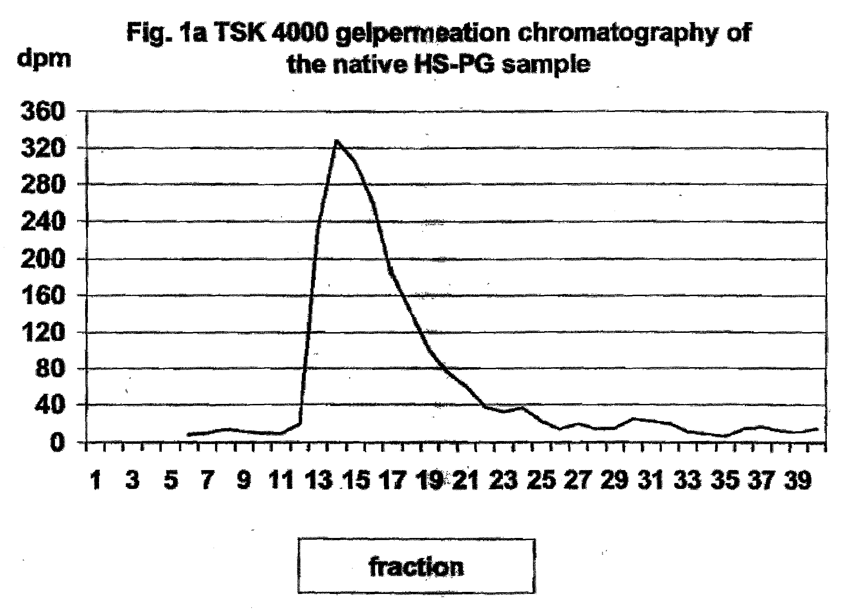

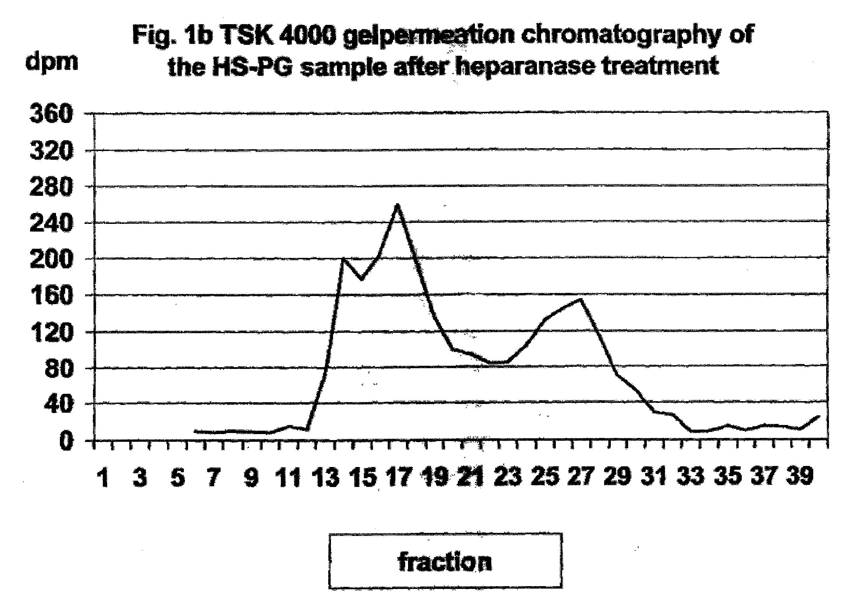

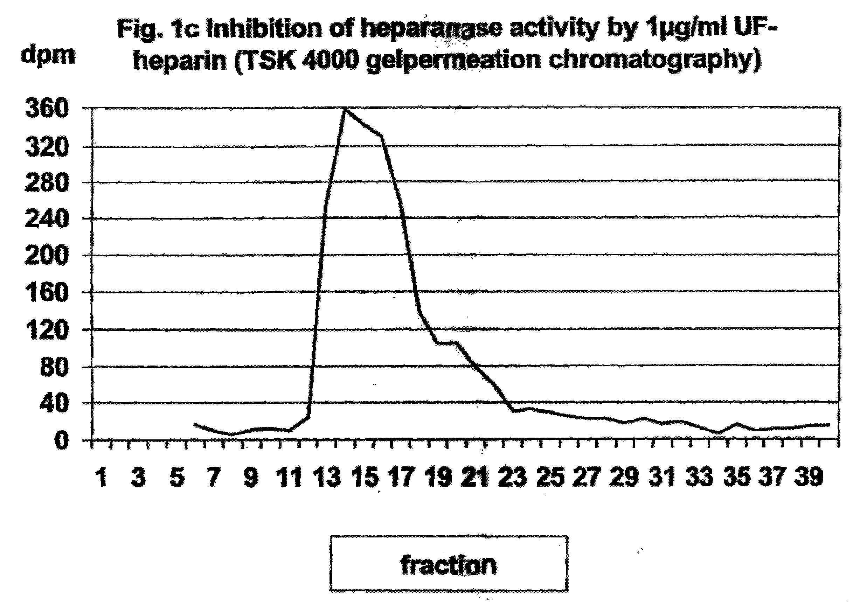

First, the heparanase assay was optimized for the purposes of this study. For practical reasons the incubation time in the degradation assay was set to 18 hours. According to the labeling efficacy and the heparan sulfate (proteoglycan) content, total counts of heparan sulfate (proteoglycans) were set to approximately 2200 dpm per sample, to allow to perform all the assays with one batch of labeled heparan sulfate (proteoglycan). The figure 1a shows a TSK 4000 gel permeation chromatography of the native sample. The figure 1b shows the shift of the molecular weight distribution of the sample that is induced by heparanase. Thereafter, the amount of heparanase was determined that allowed a degradation of about 80 % of the heparan sulfate proteoglycan (the sample contained approximately 35 % of heparan sulfate proteoglycans and about 65 % of chondroitin-/dermatan sulfate proteoglycans). Therefore, the range of about 10-80 % degradation would be relative linear and would be suitable to measure the effect of inhibitors. The figure 1c shows the effect of 1 µg/ml unfractionated heparin (UFH) on the heparanase activity with an inhibition of 97,3 %.

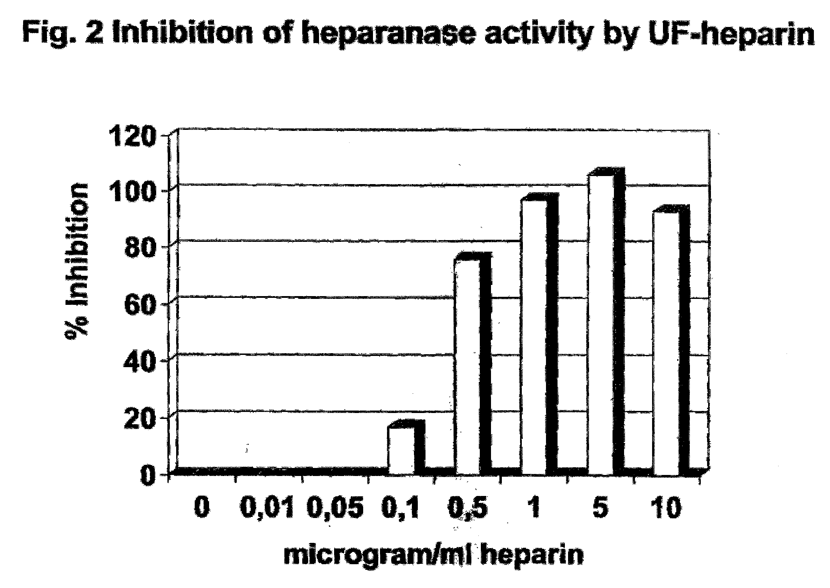

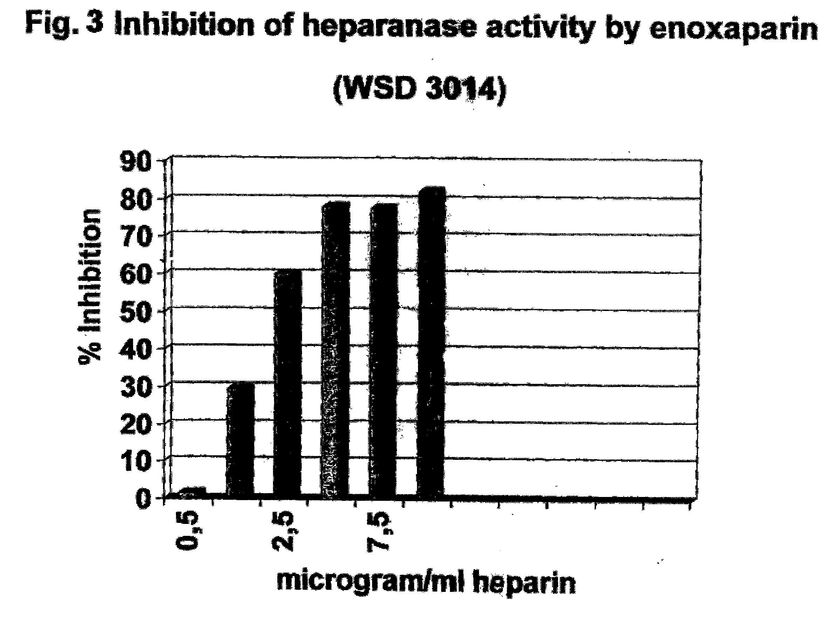

After establishment of the assay, the effect of unfractionated heparin (UFH) from porcine intestinal mucosa was tested. The figure 2 shows the dose-dependent inhibition. At concentration of 1 µg/ml of unfractionated heparin (UFH) (final concentration), a nearly complete inhibition of heparanase activity was observed. The figure 3 shows the dose-dependent inhibition of enoxaparin (WSD 3014). From this data it could be concluded, that enoxaparin shows a strong inhibitory activity of heparanase.

In general, one of ordinary skill in the art, acting in reliance upon personal knowledge and the disclosure of this application, will be able to ascertain the suitable formulation for the said chemotherapeutical agent, and in particular for docetaxel, paclitaxel, doxorubicin, cyclophasphamide, and epirubicin. Preferably suitable formulations are the marketed formulations of said chemotherapeutical agents.

In general, one of ordinary skill in the art, acting in reliance upon personal knowledge and the disclosure of this application, will also be able to ascertain the suitable formulation for enoxaparin.

For example the medicament consist of a salt (sodium or calcium preferably) or enoxaparin in the form of a composition in which the salt is combined with any other pharmaceutically compatible product, which may be inert or physiologically active. The medicament according to the invention can be used intravenously, subcutaneously, and orally. The sterile compositions for intravenous or subcutaneous administration are generally aqueous solutions. These compositions may also contain adjuvants, in particular wetting agents, tonicity agents, emulsifiers, dispersing agents and stabilizers. The sterilization can take place in several ways, for example by aseptic filtration, by incorporating sterilizing agents into the composition, or by irradiation. They may also be prepared in the form of sterile solid compositions, which can be dissolved at the time of use in sterile water or any other injectable sterile medium.

As liquid compositions for oral administration, it is possible to use solutions, suspensions, emulsions, syrups and elixirs which are pharmaceutically acceptable, containing inert diluents such as water, ethanol, glycerol, plant oils or paraffin oil. These compositions may comprise substances other than diluents, for example wetting products, sweeteners, thickeners, flavorings or stabilizers.

In general enoxaparin will be administered in therapeutically effective amounts via any of the usual and acceptable modes known in the art, either singly or in combination with one or more chemotherapeutical agent. In general chemotherapeutical agent will be administered in therapeutically effective amounts via any of the usual and acceptable modes known in the art, either singly or in combination with one or more chemotherapeutical agent. A therapeutically effective amount may wary widely depending on the severity of the disease, the age and relative health of the subject, the potency of the compound used and others factors. In general, one of ordinary skill in the art, acting in reliance upon personal knowledge and the disclosure of this application, will be able to ascertain a therapeutically effective amount of a compound for treating a given disease.

For example the dose of docetaxel would be 75 mg/m²/day, 1 hour intravenous (iv) infusion repeated 4 times at 3 weeks interval. The dose of epuribicin would be 50 mg/m²/day iv infusion repeated 4 times at 3 weeks interval.

For example the possible dose of enoxaparin may range from 10 to 40 mg injection per day, and first injection iv followed by subcutaneous (sc) repeated once daily injections during all treatment period.

In general, the physician will determine the suitable dose as a function of the age, of the weight and of all the other factors specific to the subject to be treated.

Preferably the suitable doses are the marketed doses of enoxaparin and the marketed doses of said chemotherapeutical agents.

## Claims

1. Use of enoxaparin for treating a disease linked to the modulation of heparanase activity, except when said disease is Lichen planus.

2. Use of enoxaparin in treating a disease in a mammal in which heparanase activity contributes to the pathology and/or symptoms of the disease, except when said disease is an autoimmune disease.

3. Use of enoxaparin for the manufacture of a medicament for treatment of a disease linked to modulation of heparanase activity, except when said disease is Lichen planus

4. Use of enoxaparin for the manufacture of a medicament for treatment of a disease linked to modulation of heparanse activity, except when said disease is an autoimmune disease.

5. The use according any one of claims 1 to 4, wherein said disease is linked to inhibition of heparanase activity.

6. The use according any one of claims 1 to 5, wherein said enoxaparin is in combination with one or more chemotherapeutical agent.

7. The use of claims 6, wherein said combination is a combined preparation for simultaneous, separate or sequential use.

8. The use of claim 6 or 7, wherein said chemotherapeutical agent is docetaxel, or paclitaxel, doxorubicin, epirubicin or cyclophosphamide.

9. The use of claim 6 or 7, wherein enoxaparin is in combination with docetaxel and doxorubicin.

10. The use according any one of claim 1 to 5, wherein said disease is cancer.

11. The use of claim 10, wherein said cancer is breast cancer, lung cancer, prostate cancer, colon cancer, or pancreatic cancer.

12. The use according any one of claims 1 to 11, wherein enoxaparin reduces metastasis occurrence.

13. A method for treating a disease linked to modulation of heparanase activity, except when said disease is Lichen planus, which method comprises administering to an animal a therapeutically effective amount of enoxaparin or a combination of enoxaparin and one or more chemotherapeutical agent or a pharmaceutically acceptable salt thereof, either simultaneously or separately or sequentially over time.

14. A method for treating a disease linked to modulation of heparanase activity, except when said disease is an autoimmune disease, which method comprises administering to an animal a therapeutically effective amount of enoxaparin or a combination of enoxaparin and one or more chemotherapeutical agent or a pharmaceutically salt thereof, either simultaneously or separately or sequentially over time.

15. The method of claim 13 or 14, wherein said disease is linked to inhibition of heparanase activity.

16. A method to potentiate the action of one or more chemotherapeutical agent, which comprises simultaneous, separate or sequential administration of enoxaparin.

17. The method according any one of claim 13 to 16, wherein said chemotherapeutical agent is docetaxel, paclitaxel, doxorubicin, epirubicin, or cyclophosphamide.

18. The method according any one of claim 13 to 17, wherein enoxaparin is in combination with docetaxel and doxorubicin.

19. The method of claim 13, 14 or 16, wherein said disease is cancer.
